# EUROPEAN PATENT APPLICATION

(11) **EP 3 650 040 A1**
(43) Date of publication of application: **13.05.2020**
(21) Application number: 18306460.9
(22) Date of filing: 07.11.2018
(51) Int. Cl.: A61K 39/00, A61P 31/04, A61P 17/00

(54) **VEGF INHIBITORS FOR USE FOR PREVENTING AND/OR TREATING ATOPIC DERMATITIS**

(71) Applicant: Galderma Research & Development, 06410 Biot (FR); Agency for Science, Technology and Research, Singapore 138632 (SG)
(72) Inventor: VIAL, Emmanuel, 1814 La Tour-de-Peilz (CH); HACINI-RACHINEL, Feriel, 1814 La Tour-de-Peilz (CH); JANELA, Baptiste, 259296 Singapore (SG); GINHOUX, Florent, 278178 Singapore (SG)
(74) Representative: Lavoix

(57) **Abstract**

The present invention relates to the use of a VEGF inhibitor for use for preventing and/or treating atopic dermatitis, and in particular AD associated with infection by Staphylococcus aureus.

## Description

The present invention concerns the use of VEGF inhibitors for preventing and/or treating atopic dermatitis.

Atopic dermatitis (AD) is a common, chronic, relapsing skin disease related to inflammatory dermatologic changes that are clinically characterized by pruritus and erythematosus patches and plaques with a typical morphology and distribution. See Hanifin J M. Diagnostic features of atopic dermatitis. Acta Derm Venereol 1980; 92(Suppl): 44-7. The disease manifests during infancy for most patients with AD, and the reported prevalence among children is 17% to 20% (See Levy R M, Gelfand J M, Yan A C. The epidemiology of atopic dermatitis. Clin Dermatol 2003; 21(2): 109-15). Adults are also affected by AD (from 2 to 10% of adults).

Approximately 60% AD subjects experience eruptions in the first year of life and 90% by five years of age. AD may follow a relapsing course and can be characterized by episodes of intense pruritus, lichenification, and severely dry skin as well as being susceptible to cutaneous infection. AD is often associated with elevated serum immunoglobulin (IgE) levels and/or a personal or family history of related atopic disorders, such as atopic dermatitis, allergic rhinitis or asthma.

The most common therapy for controlling AD is corticosteroid treatment; however, these therapies are not completely effective for all patients. In addition, there are concerns about side effects, especially in children, which had led patients and families to seek complementary and/or alternative treatments.

There is thus still a need for efficient atopic dermatitis treatments, which may preferably be administered via the topical route.

The present invention now offers a treatment for reaching this purpose, and offers an improved therapy for atopic dermatitis, preferably via the topical route.

Surprisingly, as shown in the examples, the inventors have discovered that, in a murine model in which *S. aureus* was injected into the skin, conventional dendritic cells 1 (cDC1) regulate the magnitude of the immune response to the bacteria *in vivo* by controlling neutrophil infiltration, at the inflamed site. The inventors have also discovered that cDC1 secrete VEGFα when active. Particularly, the injection of anti-VEGFα antibody reduces the recruitment of neutrophils.

Thus, the present invention aims to use a VEGF inhibitor for preventing and/or treating a skin infection due to *Staphylococcus aureus,* wherein it reduces the recruitment of neutrophils or prevents and/or decreases the inflammatory response to said bacteria. Indeed, said VEGF inhibitor is useful for treating and/or preventing skin bacterial infections due to *Staphylococcus aureus,* because it reduces the recruitment of neutrophils or prevents and/or decreases the inflammatory response to said bacteria.

Preferably, the present invention aims to use VEGF inhibitors for preventing and/or treating atopic dermatitis.
Indeed, targeting VEGF and inhibiting its expression, activity and/or signaling pathway, such as via the topical route (i.e. on skin), would allow controlling, and thereby reducing, the recruitment of neutrophils, thus preventing and/or decreasing the inflammatory response to *S.aureus* bacterium, and thereby the development of atopic dermatitis.

The invention thus relates to a VEGF inhibitor for use for preventing and/or treating a skin infection due to *Staphylococcus aureus* as mentioned above, preferably via the topical route.
Preferably the invention thus relates to a VEGF inhibitor for use for preventing and/or treating atopic dermatitis, preferably via the topical route.

The invention further concerns a method of preventing and/or treating a skin infection due to *Staphylococcus aureus* as mentioned above, comprising administering to a patient in need thereof a therapeutically effective amount of a VEGF inhibitor.
Preferably, the invention further concerns a method of preventing and/or treating atopic dermatitis, comprising administering to a patient in need thereof a therapeutically effective amount of a VEGF inhibitor.

By "skin infection due to *Staphylococcus aureus",* it is meant any infection of skin caused by *S.aureus* bacteria.
Said skin infection is preferably atopic dermatitis. Indeed, atopic dermatitis generally involves the prevalence of *S.aureus* bacterium in the skin. Studies have found that abnormalities in the skin barrier of persons with AD are exploited by S. aureus to trigger cytokine expression, thus aggravating the condition (Nakatsuji T, Chen TH, Two AM, Chun KA, Narala S, Geha RS, Hata TR, Gallo RL (November 2016), J of Invest Dermatol 136(11):2191-2200).
Preferably, the VEGF inhibitor is used for preventing and/or treating a skin infection due to *Staphylococcus aureus,* by reducing the recruitment of neutrophils.
Preferably, the VEGF inhibitor is used for preventing and/or treating atopic dermatitis, by reducing the recruitment of neutrophils. Preferably, the VEGF inhibitor prevents and/or decreases the inflammatory response to said bacteria. Preferably, the VEGF inhibitor prevents and/or decreases the inflammatory response to *Staphylococcus aureus.* Preferably, the VEGF inhibitor prevents and/or decreases VEGF-A-signalling on neutrophils induced by cDC1, particularly by the subset of EpCAM⁺CD59⁺Ly6D⁺ cDC1. By "VEGF-A-signalling on neutrophils", it is meant VEGF-R1 expression on neutrophils.

The subset of EpCAM⁺CD59⁺Ly6D⁺ cDC1 is a subset of dendritic cells.
Dendritic cells (DC) are professional pathogen-sensing and antigen-presenting cells (APC) that are central to the initiation and regulation of immune responses (Schlitzer et al., 2015). Conventional dendritic cells (cDCs) are innate immune cells. The term cDC refers to all DCs other than plasmacytoid DCs. In the skin, two subsets of ontogenetically-distinct and functionally-specialized conventional DC (cDC) exist: cDC1, identified in mice as expressing CD103 (integrin αE); and cDC2, expressing the CD11b (integrin αM), and SIRPα (Schlitzer and Ginhoux, 2014, Schlitzer et al., 2015, Guilliams et al., 2016).
Within the epidermis there are also Langerhans cells (LC), whose surface phenotype partially overlaps with both skin cDC (MHCII, CD207 (langerin), CD24 and CD11c) and macrophages (F4/80) (Ginhoux and Merad, 2010, Guilliams et al., 2016); however LC arise from a distinct developmental pathway and possess unique functional features (Romani et al., 2010).
Together, these APC populations sense and integrate multiple signals from the internal and external environments in order to initiate and shape optimal immune responses.
By "subset of EpCAM⁺CD59⁺Ly6D⁺ cDC1", it is meant the subset of cDC which may be present in skin and which express at least the surface markers EpCAM (epithelial cell adhesion molecule), CD59 (or MAC-inhibitory protein or membrane inhibitor of reactive lysis or MIRL) and Ly6D (or lymphocyte antigen 6 complex, locus D; or E48).

As shown in the examples, the inventors have shown that dermal cDC1 are required for the inflammatory immune response to *S.aureus* infection, and that inhibiting the VEGF signaling inhibits or decreases the neutrophil recruitment during the immune response to S. *aureus.*

VEGF (Vascular Endothelial Growth Factor) is a well-known signal protein produced by cells that stimulates vasculogenesis and angiogenesis. In mammals, the VEGF family comprises 5 members: VEGF-A (or VEGFα), placenta growth factor, VEGF-B, VEGF-C and VEGF-D.
All members of the VEGF family stimulate cellular responses by binding to tyrosine kinase receptors (the VEGFRs) on the cell surface, causing them to dimerize and become activated through transphosphorylation. The VEGF receptors have an extracellular portion consisting of 7 immunoglobulin-like domains, a single transmembrane spanning region, and an intracellular portion containing a tyrosine-kinase domain. VEGF-A binds to VEGFR-1 and VEGFR-2. VEGF-C and VEGF-D, but not VEGF-A, are ligands for a third receptor (VEGFR-3), which mediates lymphangiogenesis.

By "VEGF inhibitor" according to the present invention, it is meant any compound that inhibits or reduces VEGF biological activity. The biological activity of VEGF depends on the amount of the VEGF nucleic acid (i.e. its expression level) and/or on the amount of the VEGF protein; on VEGF interaction with its target sequences, particularly its receptor(s) (called VEGFR); or on the activation of VEGFR. Therefore, the VEGF inhibitor may reduce or inhibit VEGF expression, or reduce or inhibit VEGF interaction ability with its target sequences, particularly its receptor(s), or reduce or inhibit the activation of VEGFR. Preferably, the VEGF inhibitor is a VEGF-A inhibitor. "VEGF expression" refers to events modifying VEGF mRNA transcriptionally or post-transcriptionally, by cleavage and maturation, to provide a functional VEGF, notably any reaction which results in inhibition of VEGF mRNA processing; it also includes events modifying VEGF protein during translation, as well as post-translational modifications. As used herein, the term "target sequence" of VEGF according to the invention is a sequence to which VEGF specifically binds. Preferably, the target sequence is VEGF-R1, VEGF-R2 or VEGF-R3, more preferably VEGF-R1.

An "inhibitor of VEGF expression" refers to any compound that has a biological effect to inhibit the expression of a VEGF gene and/or the expression of a VEGF protein.
In one embodiment of the invention, said inhibitor of VEGF gene expression is a siRNA, or an antisense oligonucleotide.
Small inhibitory RNAs (siRNAs) can function as inhibitors of gene expression for use in the invention. Gene expression can be reduced with a small double stranded RNA (dsRNA), or a vector or construct causing the production of a small double stranded RNA, such that gene expression is specifically inhibited (i.e. RNA interference or RNAi). Methods for selecting an appropriate dsRNA or dsRNA-encoding vector are well known in the art for genes whose sequence is known (e.g. see U.S. Pat. Nos. 6,573,099 and 6,506,559; International Patent Nos. WO 01/36646, WO 99/32619, and WO 01/68836). Inhibitors of VEGF for use in the invention may be based on antisense oligonucleotide (ODNs) constructs. Antisense oligonucleotides, including antisense RNA molecules and antisense DNA molecules, would act to directly block the activity of VEGF by binding to VEGF mRNA and thus preventing binding leading to mRNA degradation.
For example, antisense oligonucleotides of at least about 15 bases and complementary to unique regions of the VEGF transcript sequence can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). It should be further noted that antisense oligonucleotides may be modified with phosphorothioate to prevent their in vivo hydrolysis by nucleases. Such modifications are well known in the art. Antisense oligonucleotides useful as inhibitors of VEGF can be prepared by known methods. These include techniques for chemical synthesis such as, e.g., by solid phase phosphoramidite chemical synthesis. They can also be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters.

As used herein, the terms "inhibitor of the interaction" means preventing or reducing the direct or indirect association of one or more molecules, nucleic acids, peptides, proteins. As used herein, the term "inhibitor of the interaction between VEGF and its target sequences, particularly its receptor(s)" is a molecule which can prevent the interaction between VEGF and its target sequences, particularly its receptor(s), particularly VEGF-R1, by competition or by fixing to one of the molecules.
Preferably, the VEGF inhibitor is an inhibitor of the interaction between VEGF and its receptor, preferably VEGF-R1, VEGF-R2 or VEGF-R3. Preferably the receptor is VEGF-R1. Preferably, it is a chemical molecule, a peptide, a protein or an antibody.
By "peptide", it is meant an amino acid sequence comprising from 2 to 30 amino acids. By "protein", it is meant an amino acid sequence comprising at least 31 amino acids, preferably 50 to 500 amino acids. By "antibody", it is meant a substance composed of four polypeptide chains, namely two light chains and two heavy chains.

Preferably, the VEGF inhibitor is an antibody, and preferably bevacizumab, ranibizumab or ramucirumab.

Preferably, the VEGF inhibitor is a molecule which can prevent the activation of VEGFR. Such a VEGF inhibitor is called a VEGFR tyrosine kinase inhibitor.
The test for determining whether a compound is a VEGFR tyrosine kinase inhibitor is the VEGFR kinase assay, and notably the VEGF-R2 kinase assay:
Kinase reaction is performed in a 10 µL volume in low-volume 384-well plates, NBS Corning model 4514 (black); the concentration of substrate in the assay is 200 nM, and the kinase reaction buffer consists of 50 mM HEPES pH 7.5, 0.01% BRIJ-35, 10 mM MgCl₂, and 1 mM EGTA. Kinase reactions (in the presence and in the absence of compounds) start by adding 30 ng/mL VEGF-R2, and are allowed to proceed for 1 hour at room temperature before a 10 µL preparation of EDTA (20 mM) and Tb-labeled antibody (4 nM) in TR-FRET dilution buffer are added. The final concentration of antibody in the assay well is 2 nM, and the final concentration of EDTA is 10 mM. The plate is allowed to incubate at room temperature for 60 minutes before being read on a plate reader configured for LanthaScreen™ TR-FRET.
VEGF-R1 and VEGF-R3 kinase assays would be run in a similar fashion.

Preferably, the VEGFR tyrosine kinase inhibitor is chosen from:
Sorafenib (4-[4-[[4-chloro-3-(trifluoromethyl)phenyl]carbamoylamino] phenoxy]-N-methyl-pyridine-2-carboxamide)
Sunitinib (N-(2-diethylaminoethyl)-5-[(Z)-(5-fluoro-2-oxo-1H-indol-3-ylidene)methyl]-2,4-dimethyl-1H-pyrrole-3-carboxamide)
Pazopanib (5-({4-[(2,3-Dimethyl-2H-indazol-6-yl)methylamino]pyrimidin-2-yl}amino)-2-methylbenzenesulfonamide)
Vandetanib (N-(4-bromo-2-fluorophenyl)-6-methoxy-7-[(1-methylpiperidin-4-yl)methoxy]quinazolin-4-amine)
Axitinib (N-Methyl-2-[[3-[(E)-2-pyridin-2-ylethenyl]-1H-indazol-6-yl]sulfanyl]benzamide)
Cediranib (4-[(4-fluoro-2-methyl-1H-indol-5-yl)oxy]-6-methoxy-7-[3-(pyrrolidin-1-yl)propoxy]quinazoline)
Cabozantinib (N-(4-((6,7-Dimethoxyquinolin-4-yl)oxy)phenyl)-N'-(4-fluorophenyl)cyclopropane-1,1-dicarboxamide)
Foretinib (N-(3-fluoro-4-((6-methoxy-7-(3-morpholinopropoxy)quinolin-4-yl)oxy)phenyl)-N-(4-fluorophenyl)cyclopropane-1,1-dicarboxamide)
Tivozanib (1-{2-Chloro-4-[(6,7-dimethoxyquinolin-4-yl)oxy]phenyl}-3-(5-methylisoxazol-3-yl)urea)
Lenvatinib (4-[3-Chloro-4-(cyclopropylcarbamoylamino)phenoxy]-7-methoxy-quinoline-6-carboxamide)
Lucitanib (6-({7-[(1-Aminocyclopropyl)methoxy]-6-methoxy-4-quinolinyl}oxy)-N-methyl-1-naphthamide)
Dovitinib (4-amino-5-fluoro-3-(5-(4-methylpiperazin-1-yl)-1H-benzo[d]imidazol-2-yl)quinolin-2(1H)-one)
Orantinib ((Z)-3-(2,4-dimethyl-5-((2-oxoindolin-3-ylidene)methyl)-1H-pyrrol-3-yl)propanoic acid)
Nintedanib (Methyl (3Z)-3-{[(4-{methyl[(4-methylpiperazin-1-yl)acetyl]amino}phenyl)amino](phenyl)methylidene}-2-oxo-2,3-dihydro-1H-indole-6-carboxylate)
Vatalanib (N-(4-chlorophenyl)-4-(pyridin-4-ylmethyl)phthalazin-1-amine)
Telatinib (4-(((4-((4-chlorophenyl)amino)furo[2,3-d]pyridazin-7-yl)oxy)methyl)-N-methylpicolinamide)
Motesanib (N-(3,3-Dimethyl-2,3-dihydro-1H-indol-6-yl)-2-[(pyridin-4-ylmethyl)amino]pyridine-3-carboxamide)
OSI-930 (3-((quinolin-4-ylmethyl)amino)-N-(4-(trifluoromethoxy)phenyl)thiophene-2-carboxamide)
Brivanib alaninate ((R)-(S)-1-((4-((4-fluoro-2-methyl-1H-indol-5-yl)oxy)-5-methylpyrrolo[2,1-f][1,2,4]triazin-6-yl)oxy)propan-2-yl 2-aminopropanoate)
BMS-690514 ((3R,4R)-4-amino-1-((4-((3-methoxyphenyl)amino)pyrrolo[2,1-f][1,2,4]triazin-5-yl)methyl)piperidin-3-ol)
Linifanib (1-[4-(3-amino-1H-indazol-4-yl)phenyl]-3-(2-fluoro-5-methylphenyl)urea) AEE788 (6-[4-[(4-Ethylpiperazin-1-yl)methyl]phenyl]-N-[(1R)-1-phenylethyl]-7H-pyrrolo[2,3-d]pyrimidin-4-amine)
TAK-593 (N-(5-((2-(cyclopropanecarboxamido)imidazo[1,2-b]pyridazin-6-yl)oxy)-2-methylphenyl)-1,3-dimethyl-1H-pyrazole-5-carboxamide)
CEP-11981 (13-isobutyl-4-methyl-10-(pyrimidin-2-ylamino)-1,2,4,7,8,13-hexahydro-6H-indazolo[5,4-a]pyrrolo[3,4-c]carbazol-6-one)
CP-547,632 (3-((4-bromo-2,6-difluorobenzyl)oxy)-5-(3-(4-(pyrrolidin-1-yl)butyl)ureido)isothiazole-4-carboxamide), and
Regorafenib (4-[4-({[4-Chloro-3-(trifluoromethyl)phenyl]carbamoyl}amino)-3-fluorophenoxy]-N-methylpyridine-2-carboxamide).

The VEGF inhibitor is advantageously formulated in a pharmaceutical composition, together with a pharmaceutically acceptable carrier.
"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.
The form of the pharmaceutical compositions, the route of administration, the dosage and the regimen naturally depend upon the condition to be treated, the severity of the illness, the age, weight, and sex of the patient, etc.
The pharmaceutical compositions of the invention can be formulated for a topical, oral, intraocular, intravenous, intramuscular or subcutaneous administration and the like. Preferably they are topical.
Preferably, the pharmaceutical compositions contain vehicles which are pharmaceutically acceptable for a formulation capable of being topically applied.
By topical route, the composition, in particular the pharmaceutical composition, according to the invention, can be present in all galenic forms normally used for topical administration. By way of non-limiting example of topical preparations, preparations may be mentioned in liquid, pasty or solid form and, more particularly, in the form of ointments, aqueous, aqueous-alcoholic or oily solutions, dispersions of the optionally two-phase lotion type, serum, aqueous, anhydrous or lipophilic gels, powders, soaked pads, syndets, wipes, sprays, foams, sticks, shampoos, compresses, washing bases, emulsions of liquid or semi-liquid consistency such as milk, obtained by dispersing a fatty phase in an aqueous phase (O/W) or inversely (W/O), a microemulsion, suspensions or emulsions of soft, semi-liquid or solid of white or colored cream type, gel or ointment, suspensions of microspheres or nanospheres or lipid or polymeric vesicles, or microcapsules, micro- or nanoparticles or of polymeric or gelled patches allowing a controlled release.

It is routine for those skilled in the art to adjust the nature and amount of the additional active ingredients and excipients in the pharmaceutical composition so as not to affect the desired properties thereof, notably with regard to the stability of the VEGF inhibitor according to the invention, and the route of administration considered.

The physiologically acceptable medium may comprise various excipients. By "excipient" it is meant an inert substance typically used as a diluent or carrier for the VEGF inhibitor according to the invention.

Emulsions such as oil-in-water (O/W) or water-in-oil (W/O) systems, as well as a base (vehicle or support) for the topical formulation, may be chosen so as to ensure efficacy of the active ingredients and/or to avoid allergic and irritant reactions. The compositions may comprise an emulsifier. Non-limiting examples of emulsifiers useful in this regard include glycol esters, fatty acids, fatty alcohols, fatty acid esters of glycols, fatty esters, fatty ethers, glycerine esters, propylene glycol esters, polyethylene glycol fatty acid esters, fatty acid esters of polypropylene glycol, sorbitol esters, esters of sorbitan anhydrides, copolymers of carboxylic acids, glucose esters and ethers, ethoxylated ethers, ethoxylated alcohols, alkyl phosphates, polyoxyethylene phosphate ethers, fatty acid amides, acyl lactylates, soaps and mixtures thereof. Specific non-limiting examples of emulsifiers useful in the present compositions include polyethylene glycol-20 sorbitan monolaurate (polysorbate-20), polyethylene glycol, soybean sterol, steareth-2, steareth-20, steareth-21, ceteareth-20, glucose methyl ether distearate PPG-2, ceteth-10, polysorbate-80, cetyl phosphate, potassium cetyl phosphate, diethanolamine cetyl phosphate, polysorbate-60, glyceryl stearate, PEG-100 stearate, tragacanth gum and mixtures thereof.
The lotions useful in the present compositions may be suspensions of powdered material in an aqueous or alcoholic base.
Ointments are oleaginous compositions that contain little or no water (anhydrous).
The compositions can also be in the form of gels. In this regard, the compositions may comprise a gelling agent and/or a thickener. Suitable gelling and/or thickening agents which may be useful in the present compositions include aqueous thickeners, such as neutral, anionic and cationic polymers, and mixtures thereof. Examples of polymers which may be useful in the present compositions include carboxyvinyl polymers, such as carboxypolymethylene. A preferred thickener is a carbomer, for example a Carbopol® polymer from Noveon Inc. Other examples of polymers useful in this regard include hydrophilic/hydrophobic graft copolymers, such as polymers formed as a mixture of polystyrene/microsponge/Carbopol®. Such a polymer in this respect is a dimethyl acrylamide/acrylic acid/polystyrene ethyl methacrylate copolymer, for example a copolymer of the Pharmadur® brand as available from Polytherapeutics.
Other non-limiting examples of suitable thickeners include cellulosic polymers such as arabic gum, tragacanth gum, locust bean gum, guar gum, hydroxypropylguar, xanthan gum, cellulose gum, sclerotium gum, carrageenan gum, karaya gum, cellulose, rosin, methylcellulose, hydroxyethylcellulose, hydroxypropylcellulose, hydroxymethylcellulose, hydroxypropylmethylcellulose, methylhydroxyethylcellulose, cetylhydroxyethylcellulose, carboxymethylcellulose, corn starch, hydroxypropyl phosphate starch, PEG-150/alkoxy stearyl alcohol/SMDI copolymer, PEG-180/laureth-50/TMMG copolymer, acrylic acid/acrylamidomethylpropane sulfonic acid copolymer, acrylate/C10-30 acrylate copolymer, acrylate/beheneth-25 methacrylate copolymer, acrylate/steareth-20 methacrylate copolymer, acrylate/stearth-20 copolymer, acrylate/VA copolymer, ammonium acryloyldimethyltaurate/beheneth-25 methacrylate, ammonium acryloyldimethyltaurate/VP copolymer, caprylic/capric triglyceride (and) sodium acrylate copolymer, propylene glycol alginate, dimethicone and mixtures thereof. Other thickeners and/or gelling agents, such as polyacrylic polymers, may be used.

In another embodiment, the pharmaceutical composition may be adapted for injection. The compositions may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

According to the invention, 'treatment' includes therapeutic treatment, and 'prevention' includes prophylactic or preventive treatment, wherein the object is to prevent or slow down the targeted pathologic condition or disorder. Those in need of treatment include those already with the disorder as well as those prone to have the disorder or those in whom the disorder is to be prevented. The term "treating" includes reducing, alleviating or inhibiting or eliminating the symptoms or progress of a disorder.
Preferably, an effective amount, preferably a therapeutically effective amount of the VEGF inhibitor of the invention is administered. An "effective amount" refers to an amount effective, at dosages and for periods of time necessary, to achieve the desired therapeutic or prophylactic result.
A "therapeutically effective amount" of a VEGF inhibitor of the invention may vary according to factors such as the disease state, age, sex, and weight of the individual, and the ability of the VEGF inhibitor, to elicit a desired therapeutic result. A therapeutically effective amount encompasses an amount in which any toxic or detrimental effects of the VEGF inhibitor are outweighed by the therapeutically beneficial effects. A therapeutically effective amount also encompasses an amount sufficient to confer benefit, e.g., clinical benefit.

The invention will be better understood on reading the following illustrative and non-limiting examples.

The figures are the following:

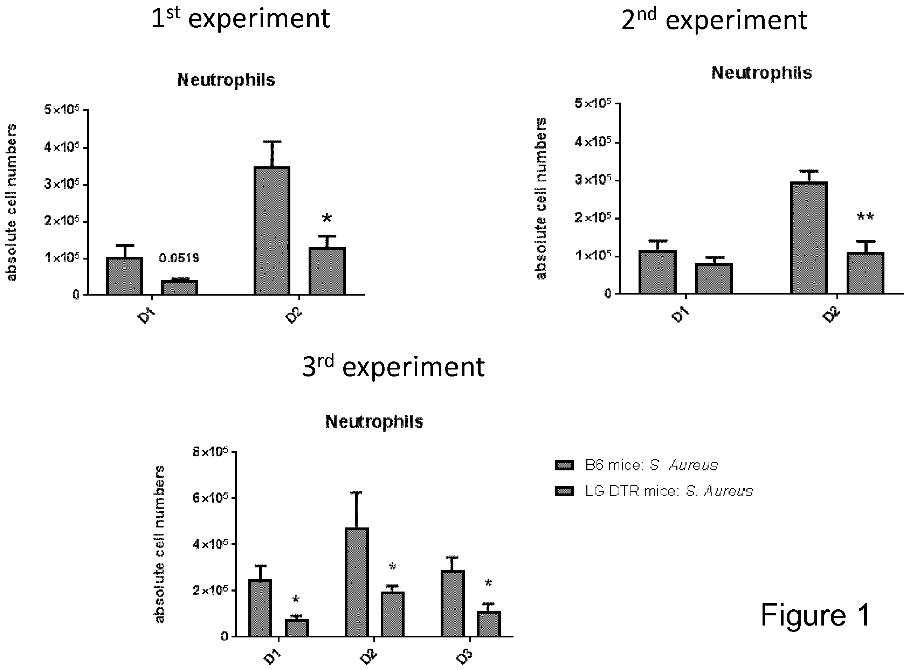

**Figure 1****: Langerin expressing cells are required for the sustained inflammatory immune response to *S. aureus.*** Ear thickness measurements and absolute neutrophil numbers in ear skin during the 2 and 3 days following intradermal *S .aureus* injection of WT B6 mice (first histograms) and LG-DTR mice injected with DT (second histograms). Shown is the mean ± SEM (*p < 0.05; **p < 0.01; ***p < 0.001).

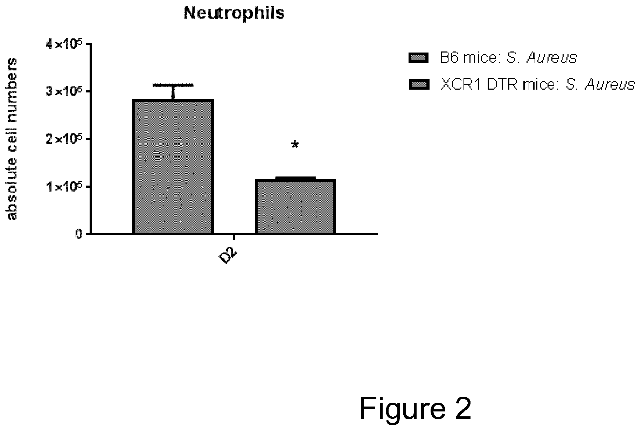

**Figure 2****: Dermal cDC1 are required for the sustained inflammatory immune response to *S. aureus.*** Absolute neutrophil numbers in ear skin at day 2 following intradermal *S. aureus* injection of WT B6 mice (first histogram) and XCR1 DTR mice injected with DT (second histogram). Shown is the mean ± SEM (*p < 0.05; **p < 0.01; ***p < 0.001).

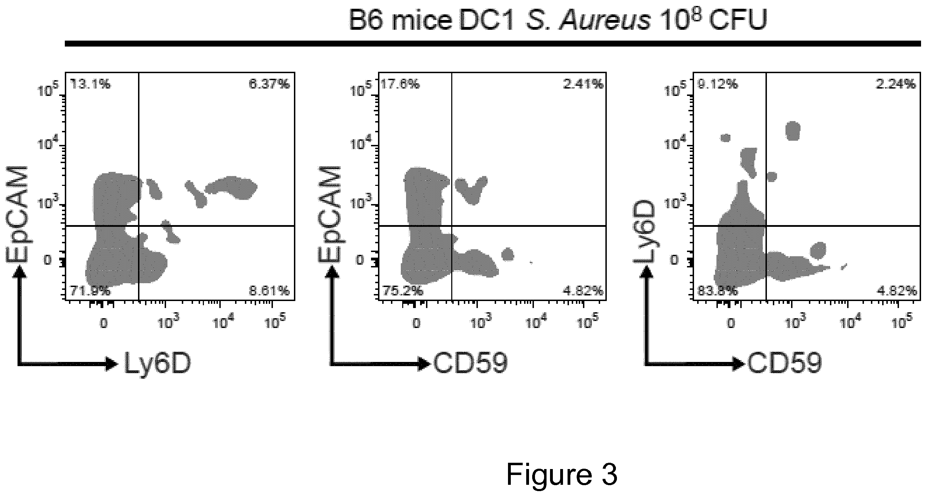

**Figure 3****: Activated dermal cDC1 are observed during immune response to *S. aureus.*** Frequency and intensity of expression of the surface proteins EPCAM, Ly6D and CD59 across the cDC1 populations from *S. aureus*-injected ear skin, measured by flow cytometry.

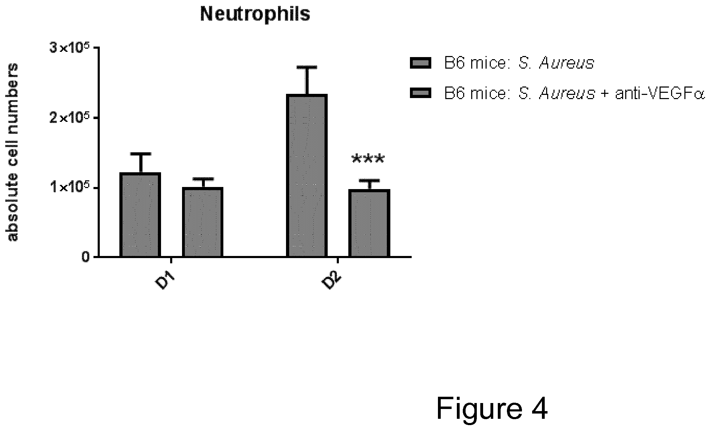

**Figure 4****: VEGF regulates the neutrophil immune wave during the immune response to *S*. *aureus.*** Ear thickness measurements and absolute neutrophil numbers in ear skin during the 2 days following intradermal *S. aureus* injection of WT B6 mice (first histograms) and WT B6 mice injected concomitantly with anti-VEGFα antibody (second histograms). Shown is the mean ± SEM (*p < 0.05; **p < 0.01; ***p < 0.001)

The first histograms are for the B6 mice injected with *S.aureus,* and the second histograms are for the B6 mice concomitantly injected with *S.aureus* and anti-VEGFα antibodies.

### Example 1: Dermal cDC1 control sustained recruitment and survival of neutrophils through VEGFα secretion in response to cutaneous S. aureus infection

### MATERIALS AND METHODS

### Mouse models

C57BL/6 (C45.2+) mice were from the Biological Resource Center, Agency for Science, Technology and Research (A*STAR), Singapore. Langerin DTR and XCR1 DTR mice were provided by B. Malissen. All experiments and procedures were approved by the Institutional Animal Care and Use Committee of the Biological Resource Center (Agency for Science, Technology and Research, Singapore) in accordance with the guidelines of the Agri-Food and Veterinary Authority and the National Advisory Committee for Laboratory Animal Research of Singapore.

### Bacterial culture and intra-dermal injection

*Staphylococcus aureus* was obtain from ATCC (ATCC® 6538™) and grown in an aerobic chamber at 37°C for 16 hours. A spectrophotometer reading OD₆₀₀ was used to identify the bacterial log growth phase. Live *S. aureus* was intradermally injected (10⁸ CFU in 20µl of PBS) into the ear of mice. For infection in Langerin-DTR and XCR1 DTR mice, diphtheria toxin (DT) was administered intraperitoneally (i.p.). Anti-VEGFα antibody (2µg, Abcam) has been intradermally injected concomitantly with *S. aureus.* Ear thickness was measured using an electronic caliper (Mitutoyo).

### Preparation of ear cell suspensions

Mouse skin cells were isolated as described previously [Ginhoux, F., M. P. Collin, et al. (2007). "Blood-derived dermal langerin+ dendritic cells survey the skin in the steady state." J Exp Med 204(13): 3133-3146]. Briefly, mouse ears were split into dorsal and ventral halves and floated in RPMI-1640 medium (Sigma) containing 1 mg/ml dispase (Invitrogen) for 60 min at 37°C to allow separation of epidermal and dermal sheets. The separated sheets were then cut into small pieces and incubated in RPMI containing 10% serum, Collagenase IV (0.2mg / ml, Roche) and DNAse I (20000U / ml, Roche) for 1 hr at 37°C. Cells were then passed through 19 G needle and filtered through a 100 µM cell strainer (BD Falcon) to obtain a homogenous cell suspension.

### Flow cytometry and fluorescence-activated cell sorting

Multi-parameter analyses and sorting of labelled cell suspensions were performed on a LSR II and FACSAria II (Becton Dickinson, San Jose, USA) respectively. Data were analysed with FlowJo software (TreeStar). Fluorochrome- or biotin- conjugated monoclonal antibodies (mAbs) against the following molecules used: mouse IA/IE (M5/114.15.2) (BD Biosciences); Ly6G (1A8), CD64 (X54-5/7.1), EpCAM (G8.8) (Biolegend), CD59 (mCD59.3), Ly6D (49-H4) (Biolegend); and CD11c (N418), CD45.2 (104), Ly6C (HK1.4), CD24 (M1/69), and CD11b (M1/70) (all from eBioscience).

### RESULTS

The inventors have analyzed the composition of the immune cell infiltrate after *S. aureus* infection. The inventors observed an increase of the ear thickness associated with a major infiltration of neutrophils into the skin during the 3 first days. In Langerin-DTR (LG DTR) mice, exposure to diphtheria toxin (DT) induces specific depletion of dermal cDC1 and Langerhans cells (LC). The inventors observed similar numbers of neutrophils, in the first 24 hours after *S. aureus* infection in the presence or absence of both cDC1 and LC, even if a trend of a lower level of neutrophils could be described. Two days after *S. aureus* injection, the absence of Langerin+ cells resulted in a significant decrease of the ear thickness associated with a major reduction of the accumulation of the number of neutrophils (Figure 1).

To distinguish the roles of LC and cDC1 in this phenomenon, the inventors have used the XCR1 cre Rosai DTR mouse model, in which injection of diphtheria toxin will induce a specific depletion of the DC1, as XCR1 is a specific/exclusive marker for the cDC1. In absence of DC1, the inventors observed a significant decrease of the ear thickness associated with a major reduction of the accumulation of the number of neutrophils at D2 post-infection (Figure 2).

During the immune response to *S. aureus,* the inventors have detected the presence of a subset of cDC1 which express the surface markers EpCAM (epithelial cell adhesion molecule), CD59 (or MAC-inhibitory protein or membrane inhibitor of reactive lysis or MIRL) and Ly6D (or lymphocyte antigen 6 complex, locus D; or E48) and which are known to have a major role in the control of the neutrophil immune response via VEGF production (Figure 3).

To understand the functional impact of VEGFα on early recruitment and regulation of neutrophils, the inventors neutralized this cytokine *in vivo* using a specific blocking antibody. After concomitant intra-dermal injection of WT B6 mice with anti-VEGFα antibodies and *S. aureus,* the inventors observed a significant reduction in ear swelling and neutrophil infiltration compared to control WT B6 mice (Figure 4).

Thus, these experiments clearly demonstrate that the use of a VEGF inhibitor, such as anti-VEGFα antibodies, may be used for preventing and/or treating a skin infection due to *S.aureus,* as it reduces the recruitment of neutrophils during the immune response to *S.aureus.*

## Claims

1. A VEGF inhibitor for use for preventing and/or treating a skin infection due to *Staphylococcus aureus* bacteria, wherein it reduces the recruitment of neutrophils or prevents and/or decreases the inflammatory response to said bacteria.

2. A VEGF inhibitor for use according to claim 1, wherein it is for preventing and/or treating atopic dermatitis.

3. The VEGF inhibitor for use according to any one of claims 1 to 2, wherein it is an inhibitor of VEGF expression, a VEGF receptor tyrosine kinase inhibitor, or an inhibitor of VEGF interaction with its target sequences, particularly its receptor(s).

4. The VEGF inhibitor for use according to any one of claims 1 to 3, wherein it is a VEGF-A inhibitor.

5. The VEGF inhibitor for use according to any one of claims 1 to 4, wherein it is an inhibitor of VEGF expression chosen from siRNA and antisense oligonucleotides.

6. The VEGF inhibitor for use according to any one of claims 1 to 4, wherein it is an inhibitor of VEGF interaction with its receptor(s) chosen from chemical molecules, peptides, proteins and antibodies.

7. The VEGF inhibitor for use according to any one of claims 1 to 4, wherein it is an inhibitor of VEGF interaction with its receptor(s) chosen from bevacizumab, ranibizumab and ramucirumab.

8. The VEGF inhibitor for use according to any one of claims 1 to 4, wherein it is a VEGFR tyrosine kinase inhibitor.

9. The VEGF inhibitor for use according to claim 8, wherein it is a VEGFR tyrosine kinase inhibitor chosen from:
Sorafenib
Sunitinib
Pazopanib
Vandetanib
Axitinib
Cediranib
Cabozantinib
Foretinib
Tivozanib
Lenvatinib
Lucitanib
Dovitinib
Orantinib
Nintedanib
Vatalanib
Telatinib
Motesanib
3-((quinolin-4-ylmethyl)amino)-N-(4-(trifluoromethoxy)phenyl)thiophene-2-carboxamide Brivanib alaninate
(3R,4R)-4-amino-1-((4-((3-methoxyphenyl)amino)pyrrolo[2,1-f][1,2,4]triazin-5-yl)methyl)piperidin-3-ol
Linifanib
6-[4-[(4-Ethylpiperazin-1-yl)methyl]phenyl]-N-[(1R)-1-phenylethyl]-7H-pyrrolo[2,3-d]pyrimidin-4-amine
N-(5-((2-(cyclopropanecarboxamido)imidazo[1,2-b]pyridazin-6-yl)oxy)-2-methylphenyl)-1,3-dimethyl-1H-pyrazole-5-carboxamide
13-isobutyl-4-methyl-10-(pyrimidin-2-ylamino)-1,2,4,7,8,13-hexahydro-6H-indazolo[5,4-a]pyrrolo[3,4-c]carbazol-6-one
3-((4-bromo-2,6-difluorobenzyl)oxy)-5-(3-(4-(pyrrolidin-1-yl)butyl)ureido)isothiazole-4-carboxamide, and
Regorafenib.

10. The VEGF inhibitor for use according to any one of claims 1 to 9, wherein it is formulated in a pharmaceutical composition, preferably a topical pharmaceutical composition.

11. The VEGF inhibitor for use according to claim 10, wherein the pharmaceutical composition is in liquid, pasty or solid form and, more particularly, in the form of ointments, aqueous, aqueous-alcoholic or oily solutions, dispersions of the optionally two-phase lotion type, serum, aqueous, anhydrous or lipophilic gels, powders, soaked pads, syndets, wipes, sprays, foams, sticks, shampoos, compresses, washing bases, emulsions of liquid or semi-liquid consistency such as milk, obtained by dispersing a fatty phase in an aqueous phase (O/W) or inversely (W/O), a microemulsion, suspensions or emulsions of soft, semi-liquid or solid of white or colored cream type, gel or ointment, suspensions of microspheres or nanospheres or lipid or polymeric vesicles, or microcapsules, micro- or nanoparticles or of polymeric or gelled patches allowing a controlled release.

12. The VEGF inhibitor for use according to any one of claims 1 to 11, wherein it prevents and/or decreases VEGF-R1 expression on neutrophils induced by cDC1, particularly by the subset of EpCAM⁺CD59⁺Ly6D⁺ cDC1.
